(19) Europäisches Patentamt
European Patent Office
Office européen des brevets

(11) Veröffentlichungsnummer: **0 195 237**
**B1**

# (12) EUROPÄISCHE PATENTSCHRIFT

(45) Veröffentlichungstag der Patentschrift: **02.11.88**

(51) Int. Cl.⁴: **C 07 D 277/34,** C 07 D 417/12, A 01 N 43/78

(21) Anmeldenummer: **86101724.2**

(22) Anmeldetag: **11.02.86**

(54) **4,5-disubstituierte 1,3-Thiazol-2-yloxyacetamide.**

(30) Priorität: **21.02.85 DE 3505902**

(43) Veröffentlichungstag der Anmeldung: **24.09.86 Patentblatt 86/39**

(45) Bekanntmachung des Hinweises auf die Patenterteilung: **02.11.88 Patentblatt 88/44**

(84) Benannte Vertragsstaaten: **AT BE CH DE FR GB IT LI NL**

(56) Entgegenhaltungen:
**EP-A-0 029 171**
**EP-A-0 039 811**
**EP-A-0 060 426**
**EP-A-0 081 730**
**EP-A-0 165 537**

(73) Patentinhaber: **BAYER AG, Konzernverwaltung RP Patentabteilung, D-5090 Leverkusen 1 Bayerwerk (DE)**

(72) Erfinder: **Förster Heinz, Dr., Am Eckbusch 47, D-5600 Wuppertal 1 (DE)**
Erfinder: **Beck, Gunther, Dr., Am Mittelberg 19, D-5090 Leverkusen (DE)**
Erfinder: **Klauke, Erich, Dr., Eichendorffweg 8, D-5063 Odenthal (DE)**
Erfinder: **Santel, Hans- Joachim, Dr., Grünstrasse 9a, D-5090 Leverkusen (DE)**
Erfinder: **Schmidt, Robert R., Dr., Im Waldwinkel 110, D-5060 Bergisch- Gladbach 2 (DE)**

**EP 0 195 237 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue 4,5-disubstituierte 1,3-Thiazol-2-yloxyacetamide, ein Verfahren zu ihrer Herstellung und ihre Verwendung als Herbizide.

Es ist bereits bekannt, daß bestimmte 4,5-disubstituierte 1,3-Thiazol-2-yloxyacetamide, wie beispielsweise das 2-(4,5-Dichlor-1,3-thiazol-2-yloxy)-N,N-diethylacetamid, herbizide, insbesondere auch selektiv-herbizide Eigenschaften besitzen (vol. z. B. EP-A-0 018 497).

Die herbizide Wirksamkeit dieser vorbekannten Verbindungen gegenüber Schadpflanzen ist jedoch ebenso wie ihre Selektivität gegenüber wichtigen Kulturpflanzen nicht immer in allen Anwendungsbereichen völlig zufriedenstellend.

Weiterhin ist bekanntgeworden, daß bestimmte substituierte Azolyl-oxycarbonsäureamide pflanzenwachstumsregulierende Eigenschaften aufweisen (vgl. EP-A-0 039 811).

Es wurden neue 4,5-disubstituierte 1,3-Thiazol-2-yloxyacetamide der allgemeinen Formel (I) gefunden,

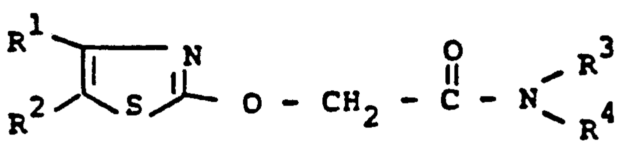

(I),

in welcher

$R^1$ für Fluor oder Chlor steht,

$R^2$ für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen (wie insbesondere Fluor, Chlor oder Brom) steht und

$R^3$ und $R^4$ unabhängig voneinander für geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen (wobei die beiden letzgenannten Reste durch Alkylreste mit 1 bis 4 Kohlenstoffatomen substituiert sein können), für jeweils geradkettiges oder verzweigtes Alkoxy, Alkoxyalkylenoxy oder Alkoxyalkyl mit jeweils 1 bis 8 Kohlenstoffatomen in den einzelnen Alkyl- bzw. Alkylenteilen, für Halogenalkyl mit 1 bis 8 Kohlenstoffatomen und 1 bis 5 Halogenatomen (insbesondere Fluor, Chlor und Brom), für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen (wobei diese Reste substituiert sein können durch: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, insbesondere Fluor Chlor und Brom, sowie Nitro), oder

$R^3$ und $R^4$ zusammen mit dem Stickstoffatom, an das sie gebunden sind, für einen gegebenenfalls einfach oder mehrfach, gleich oder verschieden substituierten, gesättigten oder ungesättigten, 5- bis 7-gliedrigen Heterocyclus stehen, (wobei diese Reste substiutiert sein können durch: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems, oder Dioxyalkylen mit 2 bis 3 Kohlenstoffatomen).

Weiterhin wurde gefunden, daß man die neuen 4,5-disubstituierten 1,3-Thiazol-2-yloxyacetamide der allgemeinen Formel (I) erhält, wenn man 4,5-disubstituierte 1,3-Thiazole der Formel (II)

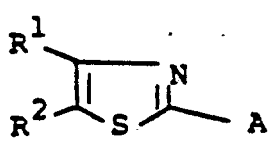

(II)

in welcher

$R^1$ und $R^2$ die oben angegebene Bedeutung haben und

A für eine elektronenanziehende Abgangsgruppierung steht,

mit Glykolsäureamiden der Formel (III)

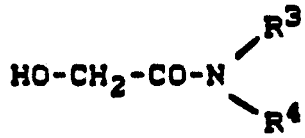

(III)

in welcher

$R^3$ und $R^4$ die oben angegebene Bedeutung haben,

gegebenenfalls in Gegenwart eines Verdünnungsmittels und gegebenenfalle in Gegenwart eines Säurebindemittels sowie gegebenenfalls in Gegenwart eines Katalysators umsetzt.

Schließlich wurde gefunden, daß die neuen 4,5-disubstituierten 1,3-Thiazol-2-yloxyacetamide der Formel (I) herbizide, insbesondere auch selektiv-herbizide Eigenschaften besitzen.

Überraschenderweise zeigen die erfindungsgemäßen 4,5-disubstituierten 1,3-Thiazol-2-yloxyacetamide der Formel (I) eine erheblich verbesserte herbizide Wirksamkeit gegen verbreitete schwer bekämpfbare Unkräuter bei vergleichbar guter Verträglichkeit gegenüber wichtigen Kulturpflanzen im Vergleich zü den aus dem Stand der Technik vorbekannten 4,5-disubstituierten 1,3-Thiazol-2-yl-oxyacetamiden, wie beispielsweise das 2-(4,5-Dichlor-1,3-thiazol-2-yloxy)-N,N-diethylacetamid, welches chemisch und wirkungsmäßig naheliegende Verbindungen sind.

Die erfindungsgemäßen 4,5-disubstituierten 1,3-Thiazol-2-yloxyacetamide sind durch die Formel (I) allgemein definiert. Bevorzugt sind Verbindungen der Formel (I), bei welchen

$R^1$ für Fluor oder Chlor steht,

$R^2$ für Methyl, Trifluormethyl, Difluormethyl, Fluormethyl, Trichlormethyl, Dichlormethyl, Chlormethyl, Fluordichlormethyl oder Difluorchlormethyl steht und

$R^3$ und $R^4$ unabhängig voneinander für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, für gegebenenfalls ein- bis dreifach, gleich oder verschieden durch Methyl oder Ethyl substituiertes Cycloalkyl bzw. Cycloalkenyl mit 5 bis 7 Kohlenstoffatomen, fur jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen, insbesondere Fluor, Brom und Chlor, für Benzyl sowie für gegebenenfalls ein- bis dreifach, gleich oder verschieden substituiertes Phenyl stehen, wobei als Substituenten besonders bevorzugt sind: Methyl, Ethyl, Methoxy, Methylthio, Trifluormethyl, Trifluormethoxy, Trifluormethylthio, Fluor, Chlor oder Nitro; oder

$R^3$ und $R^4$ zusammen mit dem Stickstoffatom, an das sie gebunden sind, für einen gegebenenfalls einbis dreifach, gleich oder verschieden substituierten Heterocyclus der Formel

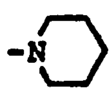 ; 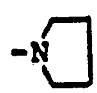 ; 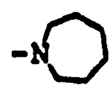 ; 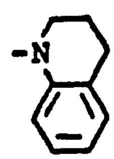

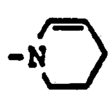 ; 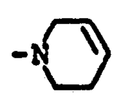

stehen, wobei als Substituenten besonders bevorzugt sind: Methyl, Ethyl und Phenyl.

Im einzelnen seien außer den bei den Herstellungsbeispielen genannten Verbindungen die folgenden Verbindungen der allgemeinen Formel (I) genannt:

**Tabelle 1**

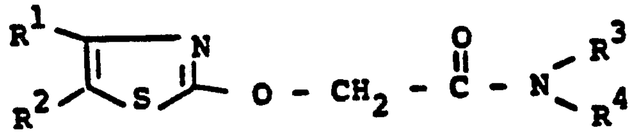

(I)

Tabelle 1

| $R^1$ | $R^2$ | $R^3$ | $R^4$ bzw. 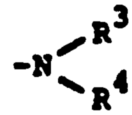 |
|---|---|---|---|
| Cl | $CF_3$ | $CH_3$ | $CH_3O-CH_2-$ |
| Cl | $CF_3$ | $CH_3$ | 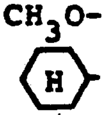 |
| Cl | $CF_3$ | $CH_3$ | 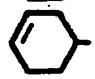 |
| Cl | $CF_3$ | $CH_3$ | $F_3C-CH_2-$ |
| Cl | $CF_3$ | $CH_3$ | 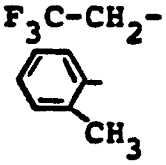 |
| Cl | $CF_3$ | $CH_3$ | 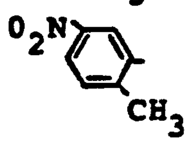 |
| Cl | $CF_3$ | $CH_3$ | 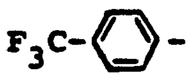 |
| Cl | $CF_3$ | $C_2H_5$ | $CH_2=CH-CH_2-$ |
| Cl | $CF_3$ | $CH_2=CH-CH_2-$ | $CH_2=CH-CH_2-$ |
| Cl | $CF_3$ | | 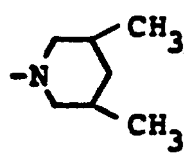 |
| Cl | $CF_3$ | | 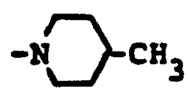 |
| Cl | $CF_3$ | | 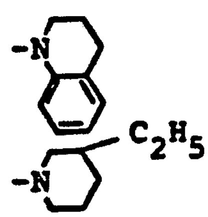 |
| Cl | $CF_3$ | | |

**Tabelle 1** (Fortsetzung)

| $R^1$ | $R^2$ | $R^3$ | $R^4$ | bzw. 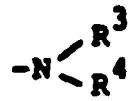 |
|---|---|---|---|---|
| Cl | $CF_3$ | $CH_3$ | $HC\equiv C-CH_2$ | |
| Cl | $CF_3$ | $CH_3O$ | $C_2H_5-CH-$<br>$CH_3$ | |
| Cl | $CF_3$ | $(CH_3)_2CHO-$ | $C_2H_5O-CH_2CH_2-O-$ | |
| Cl | $CF_3$ | $CH_3$ | 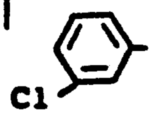 | |
| Cl | $CF_3$ | $CH_3$ | 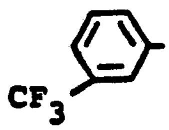 | |
| Cl | $CF_3$ | $(CH_3)_2CH-$ | $(CH_3)_2CH-O-$ | |
| Cl | $CF_3$ | $C_2H_5-CH-$<br>$CH_3$ | $CH_3O-CH_2-$ | |
| Cl | $CF_3$ | $CH_3(CH_2)_3-$ | $CH_3-(CH_2)_3-$ | |
| Cl | $CF_3$ | $C_2H_5-$ | $(CH_3)_2CH-$ | |
| Cl | $CF_3$ | $(CH_3)_2CH-$ | $C_2H_5OCH_2CH_2O-$ | |
| Cl | $CF_3$ | $CH_3$ | 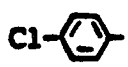 | |
| Cl | $CF_3$ | $CH_3$ | 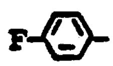 | |
| Cl | $CF_3$ | $CH_3$ | 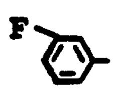 | |
| Cl | $CF_3$ | | | 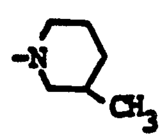 |

**Tabelle 1** (Fortsetzung)

| $R^1$ | $R^2$ | $R^3$ | $R^4$ | bzw. $-N{<}^{R^3}_{R^4}$ |
|---|---|---|---|---|
| Cl | $CF_3$ | | | 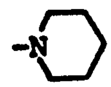 |
| Cl | $CF_3$ | | | 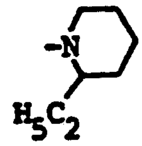 |
| Cl | $CF_3$ | | | 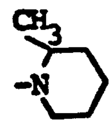 |
| Cl | $CF_3$ | $CH_3-$ | $CH_2=CH-CH_2-$ | |
| Cl | $CF_3$ | $CH_3-$ | 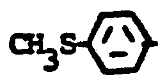 | |
| Cl | $CF_3$ | $CH_3-$ | 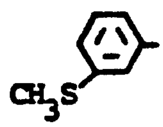 | |
| Cl | $CF_3$ | $CH_3-$ | $CH_3(CH_2)_3-$ | |
| Cl | $CF_3$ | $CH_3-$ | 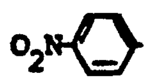 | |
| Cl | $CF_3$ | $CH_3-$ | $CH_2=CH-$ | |
| Cl | $CF_3$ | $C_2H_5-$ | $CH_2=CH-$ | |
| Cl | $CF_3$ | | | 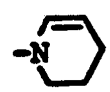 |
| Cl | $CF_3$ | | | 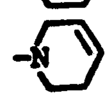 |

**Tabelle 1** (Fortsetzung)

| $R^1$ | $R^2$ | $R^3$ | $R^4$ | bzw. 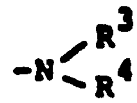 |
|---|---|---|---|---|
| F | $CF_3$ | $CH_3$ | $CH_3O-CH_2-$ | |
| F | $CF_3$ | $CH_3$ | 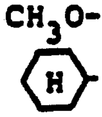 | |
| F | $CF_3$ | $CH_3$ | 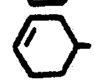 | |
| F | $CF_3$ | $CH_3$ | $F_3C-CH_2-$ | |
| F | $CF_3$ | $CH_3$ | 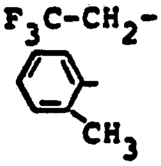 | |
| F | $CF_3$ | $CH_3$ | 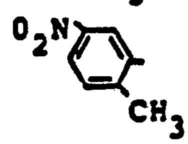 | |
| F | $CF_3$ | $CH_3$ | 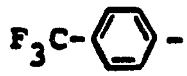 | |
| F | $CF_3$ | $C_2H_5$ | $CH_2=CH-CH_2-$ | |
| F | $CF_3$ | $CH_2=CH-CH_2-$ | $CH_2=CH-CH_2-$ | |
| F | $CF_3$ | | | 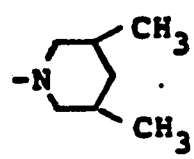 |
| F | $CF_3$ | | | 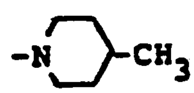 |
| F | $CF_3$ | | | 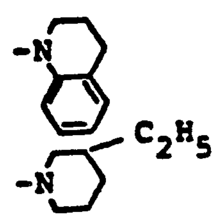 |
| F | $CF_3$ | | | |
| F | $CF_3$ | $CH_3$ | $HC\equiv C-CH_2$ | |
| F | $CF_3$ | $CH_3O$ | 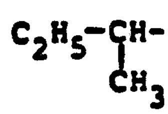 | |

**Tabelle 1** (Fortsetzung)

| $R^1$ | $R^2$ | $R^3$ | $R^4$ | bzw. $-N<^{R^3}_{R^4}$ |
|---|---|---|---|---|
| F | $CF_3$ | $(CH_3)_2CHO-$ | $C_2H_5O-CH_2CH_2-O-$ | |
| F | $CF_3$ | $CH_3$ | 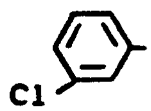 | |
| F | $CF_3$ | $CH_3$ | 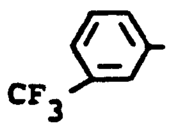 | |
| F | $CF_3$ | $(CH_3)_2CH-$ | $(CH_3)_2CH-O-$ | |
| F | $CF_3$ | $C_2H_5-CH(CH_3)-$ | $CH_3O-CH_2-$ | |
| F | $CF_3$ | $CH_3(CH_2)_3-$ | $CH_3-(CH_2)_3-$ | |
| F | $CF_3$ | $C_2H_5-$ | $(CH_3)_2CH-$ | |
| F | $CF_3$ | $(CH_3)_2CH-$ | $C_2H_5OCH_2CH_2O-$ | |
| F | $CF_3$ | $CH_3$ | 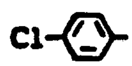 | |
| F | $CF_3$ | $CH_3$ | 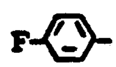 | |
| F | $CF_3$ | $CH_3$ | 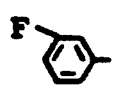 | |
| F | $CF_3$ | | | 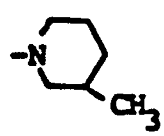 |
| F | $CF_3$ | | | 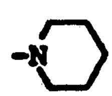 |

**Tabelle 1** (Fortsetzung)

| $R^1$ | $R^2$ | $R^3$ | $R^4$ bzw. 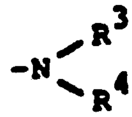 |
|---|---|---|---|
| F | $CF_3$ | | 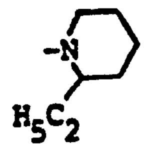 |
| F | $CF_3$ | | 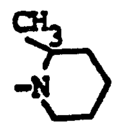 |
| F | $CF_3$ | $CH_3$- | $CH_2$=CH-$CH_2$- |
| F | $CF_3$ | $CH_3$- | 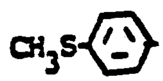 |
| F | $CF_3$ | $CH_3$- | 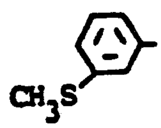 |
| F | $CF_3$ | $CH_3$- | $CH_3(CH_2)_3$- |
| F | $CF_3$ | $CH_3$- | 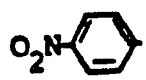 |
| F | $CF_3$ | $CH_3$- | $CH_2$=CH- |
| F | $CF_3$ | $C_2H_5$- | $CH_2$=CH- |
| F | $CF_3$ | | 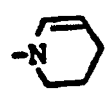 |
| F | $CF_3$ | | 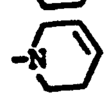 |
| F | $CHF_2$ | $CH_3$ | $CH_3O$-$CH_2$- |
| F | $CHF_2$ | $CH_3$ | 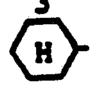 |
| F | $CHF_2$ | $CH_3$ | 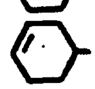 |
| F | $CHF_2$ | $CH_3$ | $F_3C$-$CH_2$- |

**Tabelle 1** (Fortsetzung)

| $R^1$ | $R^2$ | $R^3$ | $R^4$ | bzw. 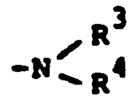 |
|---|---|---|---|---|
| F | $CHF_2$ | $CH_3$ | 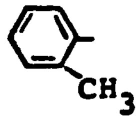 | |
| F | $CHF_2$ | $CH_3$ | 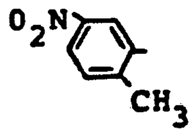 | |
| F | $CHF_2$ | $CH_3$ | 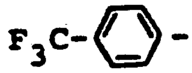 | |
| F | $CHF_2$ | $C_2H_5$ | $CH_2=CH-CH_2-$ | |
| F | $CHF_2$ | $CH_2=CH-CH_2-$ | $CH_2=CH-CH_2-$ | |
| F | $CHF_2$ | | | 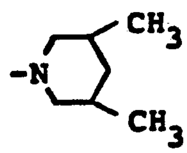 |
| F | $CHF_2$ | | | 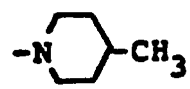 |
| F | $CHF_2$ | | | 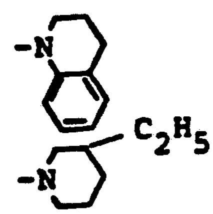 |
| F | $CHF_2$ | | | |
| F | $CHF_2$ | $CH_3$ | $HC\equiv C-CH_2$ | |
| F | $CHF_2$ | $CH_3O$ | $C_2H_5-\underset{CH_3}{\underset{\mid}{CH}}-$ | |
| F | $CHF_2$ | $(CH_3)_2CHO-$ | $C_2H_5O-CH_2CH_2-O-$ | |
| F | $CHF_2$ | $CH_3$ | 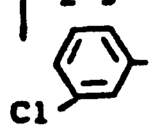 | |

**Tabelle 1** (Fortsetzung)

| $R^1$ | $R^2$ | $R^3$ | $R^4$ bzw. $-N{<}^{R^3}_{R^4}$ |
|---|---|---|---|
| F | $CHF_2$ | $CH_3$ | 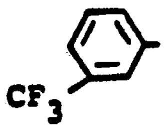 |
| F | $CHF_2$ | $(CH_3)_2CH-$ | $(CH_3)_2CH-O-$ |
| F | $CHF_2$ | $C_2H_5-CH(CH_3)-$ | $CH_3O-CH_2-$ |
| F | $CHF_2$ | $CH_3(CH_2)_3-$ | $CH_3-(CH_2)_3-$ |
| F | $CHF_2$ | $C_2H_5-$ | $(CH_3)_2CH-$ |
| F | $CHF_2$ | $(CH_3)_2CH-$ | $C_2H_5OCH_2CH_2O-$ |
| F | $CHF_2$ | $CH_3$ | 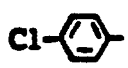 |
| F | $CHF_2$ | $CH_3$ | 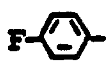 |
| F | $CHF_2$ | $CH_3$ | 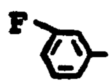 |
| F | $CHF_2$ | | 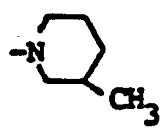 |
| F | $CHF_2$ | | 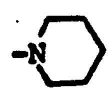 |
| F | $CHF_2$ | | 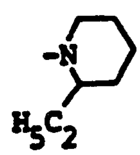 |
| F | $CHF_2$ | | 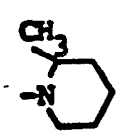 |

**Tabelle 1** (Fortsetzung)

| $R^1$ | $R^2$ | $R^3$ | $R^4$ bzw. | $-N<^{R^3}_{R^4}$ |
|---|---|---|---|---|
| F | $CHF_2$ | $CH_3-$ | $CH_2=CH-CH_2-$ | |
| F | $CHF_2$ | $CH_3-$ | 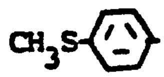 | |
| F | $CHF_2$ | $CH_3-$ | 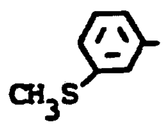 | |
| F | $CHF_2$ | $CH_3-$ | $CH_3(CH_2)_3-$ | |
| F | $CHF_2$ | $CH_3-$ | 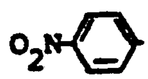 | |
| F | $CHF_2$ | $CH_3-$ | $CH_2=CH-$ | |
| F | $CHF_2$ | $C_2H_5-$ | $CH_2=CH-$ | |
| F | $CHF_2$ | | | 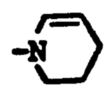 |
| F | $CHF_2$ | | | 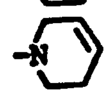 |
| F | $CH_2F$ | $CH_3$ | $CH_3O-CH_2-$ | |
| F | $CH_2F$ | $CH_3$ | 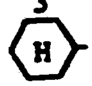 | |
| F | $CH_2F$ | $CH_3$ | 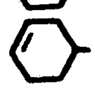 | |
| F | $CH_2F$ | $CH_3$ | $F_3C-CH_2-$ | |
| F | $CH_2F$ | $CH_3$ | 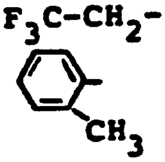 | |
| F | $CH_2F$ | $CH_3$ | 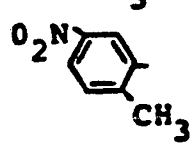 | |
| F | $CH_2F$ | $CH_3$ | 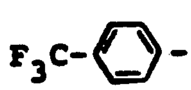 | |

**Tabelle 1** (Fortsetzung)

| $R^1$ | $R^2$ | $R^3$ | $R^4$ | bzw. $-N\begin{smallmatrix}R^3\\R^4\end{smallmatrix}$ |
|---|---|---|---|---|
| F | $CH_2F$ | $C_2H_5$ | $CH_2=CH-CH_2-$ | |
| F | $CH_2F$ | $CH_2=CH-CH_2-$ | $CH_2=CH-CH_2-$ | |
| F | $CH_2F$ | | | 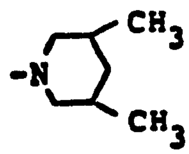 |
| F | $CH_2F$ | | | 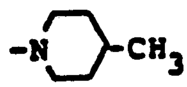 |
| F | $CH_2F$ | | | 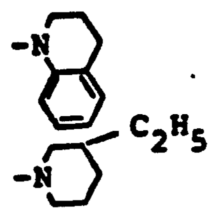 |
| F | $CH_2F$ | | | |
| F | $CH_2F$ | $CH_3$ | $HC\equiv C-CH_2$ | |
| F | $CH_2F$ | $CH_3O$ | $C_2H_5-CH(CH_3)-$ | |
| F | $CH_2F$ | $(CH_3)_2CHO-$ | $C_2H_5O-CH_2CH_2-O-$ | |
| F | $CH_2F$ | $CH_3$ | 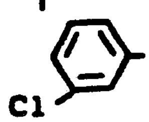 | |
| F | $CH_2F$ | $CH_3$ | 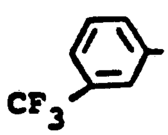 | |
| F | $CH_2F$ | $(CH_3)_2CH-$ | $(CH_3)_2CH-O-$ | |
| F | $CH_2F$ | $C_2H_5-CH(CH_3)-$ | $CH_3O-CH_2-$ | |

**Tabelle 1** (Fortsetzung)

| $R^1$ | $R^2$ | $R^3$ | $R^4$ | bzw. 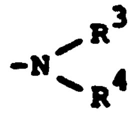 |
|---|---|---|---|---|
| F | $CH_2F$ | $CH_3(CH_2)_3-$ | $CH_3-(CH_2)_3-$ | |
| F | $CH_2F$ | $C_2H_5-$ | $(CH_3)_2CH-$ | |
| F | $CH_2F$ | $(CH_3)_2CH-$ | $C_2H_5OCH_2CH_2O-$ | |
| F | $CH_2F$ | $CH_3$ | 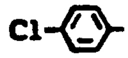 | |
| F | $CH_2F$ | $CH_3$ | 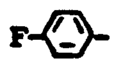 | |
| F | $CH_2F$ | $CH_3$ | 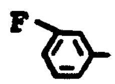 | |
| F | $CH_2F$ | | | 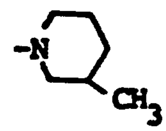 |
| F | $CH_2F$ | | | 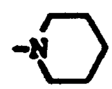 |
| F | $CH_2F$ | | | 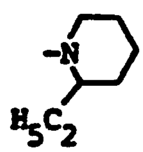 |
| F | $CH_2F$ | | | 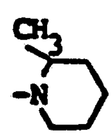 |
| F | $CH_2F$ | $CH_3-$ | $CH_2=CH-CH_2-$ | |
| F | $CH_2F$ | $CH_3-$ | 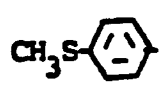 | |
| F | $CH_2F$ | $CH_3-$ | 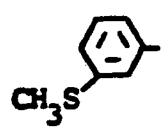 | |

**Tabelle 1** (Fortsetzung)

| $R^1$ | $R^2$ | $R^3$ | $R^4$ bzw. | 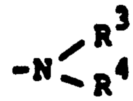 |
|---|---|---|---|---|
| F | $CH_2F$ | $CH_3$- | $CH_3(CH_2)_3$- | |
| F | $CH_2F$ | $CH_3$- | 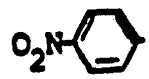 | |
| F | $CH_2F$ | $CH_3$- | $CH_2$=CH- | |
| F | $CH_2F$ | $C_2H_5$- | $CH_2$=CH- | |
| F | $CH_2F$ | | | 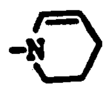 |
| F | $CH_2F$ | | | 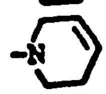 |
| Cl | $CHF_2$ | $CH_3$ | $CH_3O$-$CH_2$- | |
| Cl | $CHF_2$ | $CH_3$ | 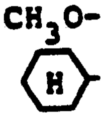 | |
| Cl | $CHF_2$ | $CH_3$ | 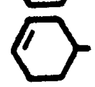 | |
| Cl | $CHF_2$ | $CH_3$ | $F_3C$-$CH_2$- | |
| Cl | $CHF_2$ | $CH_3$ | 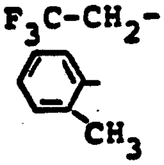 | |
| Cl | $CHF_2$ | $CH_3$ | 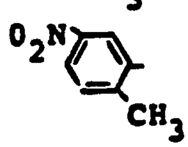 | |
| Cl | $CHF_2$ | $CH_3$ | 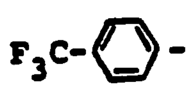 | |
| Cl | $CHF_2$ | $C_2H_5$ | $CH_2$=CH-$CH_2$- | |
| Cl | $CHF_2$ | $CH_2$=CH-$CH_2$- | $CH_2$=CH-$CH_2$- | |
| Cl | $CHF_2$ | | | 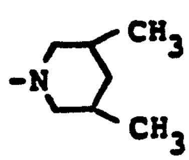 |

**Tabelle 1** (Fortsetzung)

| $R^1$ | $R^2$ | $R^3$ | $R^4$ | bzw. $-N{<}^{R^3}_{R^4}$ |
|---|---|---|---|---|
| Cl | $CHF_2$ | | | 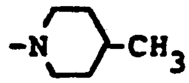 |
| Cl | $CHF_2$ | | | 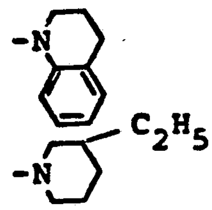 |
| Cl | $CHF_2$ | | | |
| Cl | $CHF_2$ | $CH_3$ | $HC\equiv C-CH_2$ | |
| Cl | $CHF_2$ | $CH_3O$ | $C_2H_5-CH(CH_3)-$ | |
| Cl | $CHF_2$ | $(CH_3)_2CHO-$ | $C_2H_5O-CH_2CH_2-O-$ | |
| Cl | $CHF_2$ | $CH_3$ | 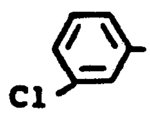 | |
| Cl | $CHF_2$ | $CH_3$ | 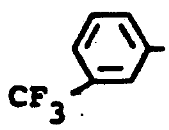 | |
| Cl | $CHF_2$ | $(CH_3)_2CH-$ | $(CH_3)_2CH-O-$ | |
| Cl | $CHF_2$ | $C_2H_5-CH(CH_3)-$ | $CH_3O-CH_2-$ | |
| Cl | $CHF_2$ | $CH_3(CH_2)_3-$ | $CH_3-(CH_2)_3-$ | |
| Cl | $CHF_2$ | $C_2H_5-$ | $(CH_3)_2CH-$ | |
| Cl | $CHF_2$ | $(CH_3)_2CH-$ | $C_2H_5OCH_2CH_2O-$ | |

**Tabelle 1** (Fortsetzung)

| $R^1$ | $R^2$ | $R^3$ | $R^4$ bzw. | 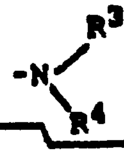 |
|---|---|---|---|---|
| Cl | $CHF_2$ | $CH_3$ | 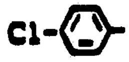 | |
| Cl | $CHF_2$ | $CH_3$ | 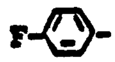 | |
| Cl | $CHF_2$ | $CH_3$ | 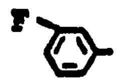 | |
| Cl | $CHF_2$ | | | 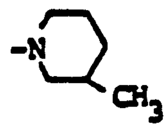 |
| Cl | $CHF_2$ | | | 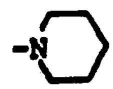 |
| Cl | $CHF_2$ | | | 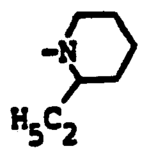 |
| Cl | $CHF_2$ | | | 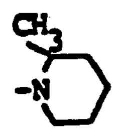 |
| Cl | $CHF_2$ | $CH_3-$ | $CH_2=CH-CH_2-$ | |
| Cl | $CHF_2$ | $CH_3-$ | 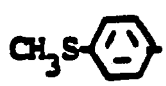 | |
| Cl | $CHF_2$ | $CH_3-$ | 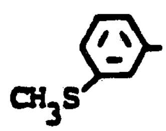 | |
| Cl | $CHF_2$ | $CH_3-$ | $CH_3(CH_2)_3-$ | |
| Cl | $CHF_2$ | $CH_3-$ | 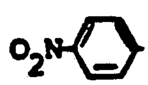 | |
| Cl | $CHF_2$ | $CH_3-$ | $CH_2=CH-$ | |
| Cl | $CHF_2$ | $C_2H_5-$ | $CH_2=CH-$ | |
| Cl | $CHF_2$ | | | 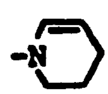 |
| Cl | $CHF_2$ | | | 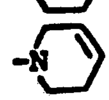 |

Verwendet man beispielsweise 2-Chlor-4-fluor-5-trifluormethyl-1,3-thiazol und Glykolsäure-N-methylanilid als Ausgangsstoffe, so läßt sich der Reaktionsablauf des erfindungsgemäßen Verfahrens durch das folgende Formelschema darstellen:

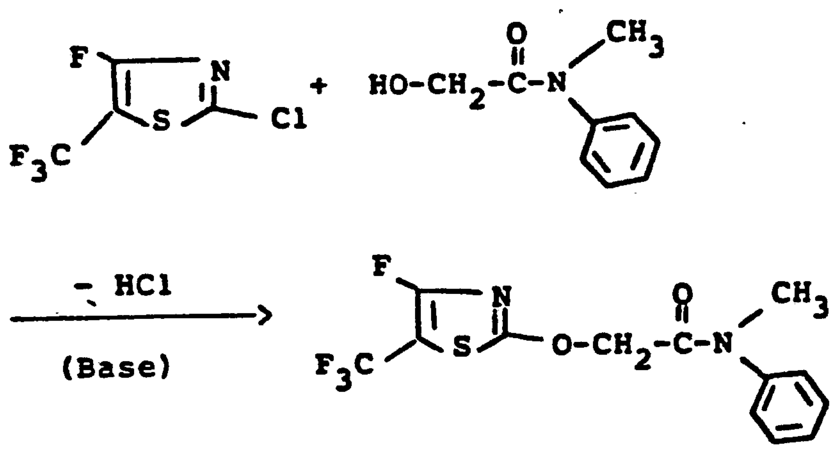


Die zur Durchführung des erfindungsgemäßen Verfahrens als Ausgangsstoffe benötigten 4,5-disubstituierten 1,3-Thiazole sind durch die Formel (II) allgemein definiert. Bevorzugt sind Verbindungen der Formel (II), bei welchen $R^1$ und $R^2$ für diejenigen Reste stehen, die bereits im Zusammenhang mit der Beschreibung der erfindungsgemäßen Stoffe der Formel (I) als bevorzugt für diese Substituenten genannt wurden: A steht vorzugsweise für Halogen, insbesondere Chlor oder Fluor.

Die 4,5-disubstituierten 1,3-Thiazole der Formel (II) sind noch nicht bekannt (Ausnahme ist das 2,4-Dichlor-5-trifluormethyl-1,3-thiazol, vgl. J. Heterocycl. Chem. 13, 1297 - 1304 (1976), welches jedoch bisher noch nicht isoliert wurde).

Noch nicht bekannte 4,5-disubstituierte 1,3-Thiazole der Formel (IIa),

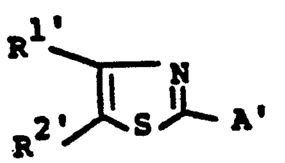

(IIa)

in welcher

A', $R^{1'}$ und $R^{2'}$ die gleiche Bedeutung haben wie die oben angegebenen Substituenten A, $R^1$ und $R^2$, wobei jedoch nicht gleichzeitig A' und $R^{1'}$ für Chlor stehen, wenn $R^{2'}$ für Trifluormethyl steht,

erhält man, wenn man Thiazolidindione der Formel (IV),

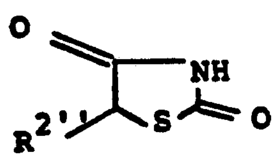

(IV)

in welcher

$R^{2''}$ für Alkyl, insbesondere für Methyl steht, mit Phosphoroxychlorid gegebenenfalls in Gegenwart eines Katalysators wie beispielsweise N,N-Dimethylformamid bei Temperaturen zwischen +50°C und +120°C umsetzt und gegebenenfalls in einer 2. Stufe die so erhalten 2,4-Dichlor-1,3-thiazole der Formel (IIb),

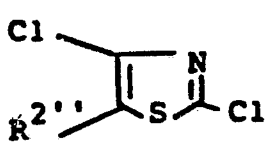

(IIb)

in welcher

$R^{2''}$ die oben angegebene Bedeutung hat,

mit Chlor gegebenenfalls in Gegenwart eines Verdünnungsmittels wie beispielsweise Tetrachlorkohlenstoff oder Phosphoroxychlorid bei Temperaturen zwischen 50°C und 250°C umsetzt und gegebenenfalls in einer 3. Stufe die so erhältlichen 5-Chloralkyl-2,4-dichlor-1,3-thiazole der Formel (IIc),

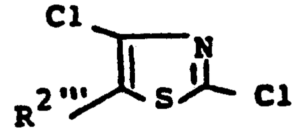

(IIc)

in welcher
$R^{2'''}$ für Chloralkyl, insbesondere für Chlormethyl, Dichlormethyl oder Trichlormethyl steht,
mit Fluorwasserstoffsäure oder einem Alkalimetallfluorid wie beispielsweise Kaliumfluorid gegebenenfalls in Gegenwart eines Verdünnungsmittels, wie beispielsweise Tetramethylensulfon, gegebenenfalls unter Druck bei Temperaturen zwischen +40°C und +200°C fluoriert.

Die Thiazolidindione der Formel (IV) sind bekannt (vgl. z. B. J. prakt. Chemie (2) 123, 114 - 121 (1931)), oder lassen sich nach bekannten Verfahren in analoger Weise erhalten.

Die zur Durchführung die erfindungsgemäßen Verfahrens weiterhin als Ausgangsstoffe benötigten Glykolsäureamide sind durch die Formel (III) allgemein definiert. In dieser Formel (III) stehen $R^3$ und $R^4$ vorzugsweise für diejenigen Reste, die schon bei der Beschreibung der erfindungsgemäßen Stoffe der Formel (I) als bevorzugt für diese Substituenten genannt wurden. Die Glykolsäureamide der Formel (III) sind ebenfalls bekannt (vgl. z. B. DE-OS-2 904 190, EP-OS-5 501, EP-OS-29 171, DE-OS-3 038 598, DE-OS-3 244 956).

Als Verdünnungsmittel für das erfindungsgemäße Verfahren kommen organische oder anorganische Lösungsmittel infrage. Bevorzugt sind Kohlenwasserstoffe, wie Toluol oder Cyclohexan, Halogenkohlenwasserstoffe, wie Methylenchlorid, Chloroform, Dichlorethan oder Chlorbenzol, Ketone, wie Aceton oder Methylisobutylketon, Ether, wie Diethylether, Diisopropylether oder Methyl-t-butylether, Alkohole, wie Methanol, Ethanol oder Isopropamol, Amide, wie Dimethylformamid oder Dimethylacetamid, Sulfoxide, wie Dimethylsulfoxid, Wasser oder wäßrige Salzlösungen.

Als Salze verwendet man hierbei vorzugeweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z. B. Natrium-, Kalium-, Magnesium- und Calciumoxid, Hydroxide, wie z. B. Natrium-, Kalium-, Magnesium- und Calciunhydroxid und/oder Carbonat, wie z. B. Natrium-, Kalium-, Magnesium- und Calciumcarbonat verwendet.

Der Zusatz von 0,01 bis 10 Gew.-% (bezogen auf eingesetztes Glykolsäureamid der Formel (III)) eines Phasentransferkatalysators mag sich in einigen Fällen als vorteilhaft erweisen. Als Beispiele für solche Katalysatoren seien genannt:
Tetrabutylammoniumchlorid, Tetrabutylammoniumbromid, Tributyl-methylphosphoniumbromid, Trimethyl-$C_{13}$/-$C_{15}$-alkyl-ammoniumchlorid, Dibenzyl-dimethyl-ammoniummethylsulfat, Dimethyl-$C_{12}$/$C_{14}$-alkyl-benzylammoniumchlorid, Tetrabutylammoniumhydroxid, 18-Krome-6, Triethylbenzylammoniumchlorid, Trimethylbenzylammomiumchlorid, Tetraethylammoniumbromid.

Die Reaktionstemperaturen können bei dem erfindungsgemäßen Verfahren innerhalb eines größeren Bereiches variiert werden. Sie liegen im allgemeinen zwischen -50°C und +100°C, vorzugsweise zwischen -20°C und +100°C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungegemäßen Verfahrens setzt man pro Mol an 4,5-disubstituiertem 1,3-Thiazol der Formel (II) im allgemeinen 0,1 bis 10 Mol, vorzugsweise 0,8 bis 1,2 Mol, an Glykolsäureamid der Formel (III) und 0,5 bis 10 Mol, vorzugsweise 0,5 bis 3 Mol, am Base ein. Die Zugabereihenfolge der Reaktanten kann beliebig vertauscht werden, es können auch alle Komponenten Bleichzeitig in das Reaktionsgefäß eindosiert werden. Die Reaktionsführung kann kontinuierlich oder diskontinuierlich gestaltet werden. Die Aufarbeitung erfolgt in üblicher Art und Weise.

Die erfindungsgemäßen Wirkstoffe können als Defoliants, Desiccants, Krautabtötungsmittel und insbesondere als Unkrautvernichtungemittel verwendet werden. Unter Unkraut im weitesten Sinne sind alle Pflanzen zu verstehen, die am Orten aufwachsen, wo sie unerwünscht sind. Ob die erfindungsgemäßen Stoffe als totale oder selektive Herbizide wirken, hängt im wesentlichen von der angewendeten Menge ab.

Die erfindungegemäßen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Circium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Polanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Eimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum,

Saccharum, Ananas, Asparagus, Allium.

Die Verwendung der erfindungsgemäßen Wirkstoffe ist jedoch keineswege auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen, z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungegemäßen Wirkstoffe neben einer hervorragenden Wirkung gegenüber Schadpflanzen auch eine gute Verträglichkeit gegenüber wichtigen Kulturpflanzen und können daher als seliktive Unkrautbekämpfungsmittel in dikotylen Kulturen, wie Sojabohnen, Baumwolle, Zuckerrüben und anderen eingesetzt werden.

Darüber hinaus weisen die erfindungegemäßen Wirkstoffe in entsprechenden Aufwandmengen auch eine starke fungizide und pflanzenwachstumsregulierende Wirkung auf.

Dabei lassen sich die erfindungegemäßen Wirkstoffe mit besonders gutem Erfolg zur Bekämpfung von Reiskrankheiten wie beispeilsweise gegen den Erreger der Reisfleckenkrankheit (Pyricularia oryzae) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in poylmeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfelösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen infrage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sagemehl, Kokosnußschalen, Maiskolben und Tabakstengel; als Emulgier- und/oder schaumerzeugende Mittel kommen infrage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate: als Dispergiermittel kommen infrage: z. B. Lignin-Sulfitablaugen und Methylcellulose.

Es können in den Formulierungen Haftmittle wie Carboxymethylcellulose, natürliche und synthetische pulverige, körnige oder latexformige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 Gew.-%.

Bei der Verwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen und Tankmischung möglich sind.

Für die Mischung kommen bekannte Herbizide wie z. B. 1-Amino-6-ethylthio-3-(2,2-dimethylpropyl)-1,3,5-triazin-2,4(1H,3H)-dion oder N-(2-Benzthiazolyl)-N,N'-dimethylharnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,2-dimethylethyl)-3-methylthio-1,2,4-triazin-5(4H)-on zur Unkrautbekämpfungin Sojabohnen, infrage. Auch Mischungen mit N,N-Dimethyl-N'-(trifluormethylphenyl)-harnstoff, Chloressigsäure-N-(methoxy-methyl)-2,6-diethylanilid, 2-Ethyl-6-methyl-N-(1-methyl-2-methoxyethyl)-chloracetanilid; 2,6-Dinitro-4-trifluormethyl-N,N-dipropylanilin; 5-Amino-4-chlor-2-phenyl-2,3-dihydro-3-oxo-pyridazin; N,N-Diisopropyl-S-(2,3,3-trichlorallyl)-thiolcarbamat; 2-Ethoxy-2,3-dihydro-3,3-dimethyl-5-benzofuranyl-methansulfonat; 1-Isobutylaminocarbonyl-2-imidazolidinon; N-Cyclohexyl-N,S-diethyl-thiolcarbamat; 3-Cyclohexyl-5,6-trimethylen-uracil, mit weiteren

Heteroaryloxyacetamiden oder Aryl- bzw. Heteroaryloxy-phenoxy-propionsäuren sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewüneshten Effektes ab. Im allgemeinen liegen die Aufwandmengen bei der Anwendung als Herbizide zwischen 0,01 une 10 kg Wirkstoff pro Hektar Bodenfläche, vorzugeweise zwischen 0,05 und 5 kg pro ha.

Die nachfolgenden Beispiele dienen zur weiteren Erläuterung der Erfindung.

**Herstellungsbeispiele:**

**Beispiel 1**

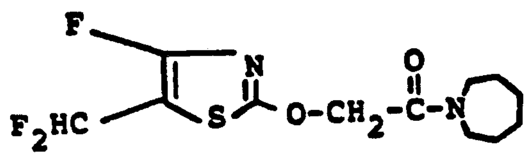

Zu 7,9 g (0,05 Mol) Hydroxyessigeäure-N,N-hexamethylenamid und 3,1 g (0,05 Mol) Kaliumhydroxid in 100 ml Isopropanol tropft man bei -20°C langsam unter Rühren 8,5 g (0,05 Mol) 2-Chlor-4-fluor-5-difluormethyl-1,3-thiazol in 10 ml Acetonitril und rührt nach beendeter Zugabe für weitere 12 Stunden bei -20°C. Wenn sich im Dünnschichtchromatogramm kein Ausgangsprodukt mehr nachweisen läßt, gießt man die Reaktionsmischung in Wasser, saugt das kristalline Produkt ab und wäscht mit Wasser und wenig kaltem Ligroin nach. Man erhält 12 g (80 % der Theorie) an 2-(4-Fluor-5-difluormethylthiazol-2-yloxy)-essigsäure-N,N-hexamethylenamid vom Schmelzpunkt 66°C.

In entsprechender Weise und gemäß den allgemeinen Herstellungsangaben erhält man die folgenden Verbindungen der allgemeinen Formel (I) - vgl. Tabelle 2:

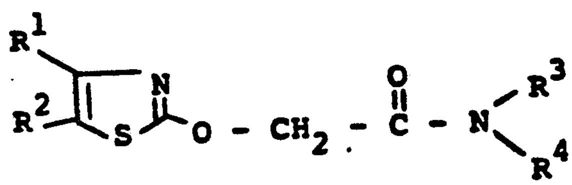

(I)

(Fallen die Substanzen als Öle an, werden sie, wie allgemein üblich, durch Extraktion mit einem organischen Lösungsmittel aus der wäßrigen Mischung isoliert).

Tabelle 2

| Bsp. Nr. | $R^1$ | $R^2$ | 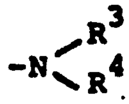 | physikalische Daten |
|---|---|---|---|---|
| 2 | Cl | $CH_3$ | 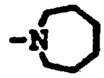 | Fp:61°C |
| 3 | Cl | $CH_3$ | 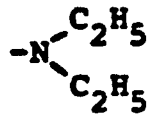 | Fp:42°C |
| 4 | Cl | $CH_3$ | 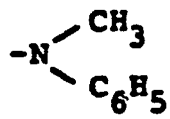 | Fp:82°C |
| 5 | Cl | $CHCl_2$ | 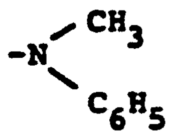 | Fp:94°C |
| 6 | Cl | $CF_2Cl$ | 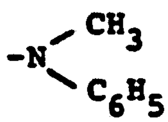 | Fp:84°C |
| 7 | Cl | $CF_2Cl$ | 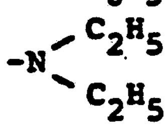 | $n_D^{20}=1,5060$ |
| 8 | Cl | $CF_2Cl$ | 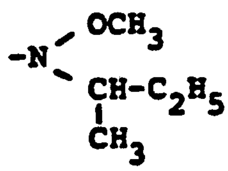 | $n_D^{20}=1,4950$ |
| 9 | Cl | $CHF_2$ | 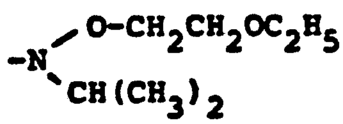 | $n_D^{20}=1,4891$ |
| 10 | Cl | $CHF_2$ | 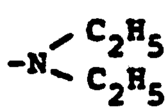 | Fp:64°C |
| 11 | Cl | $CHF_2$ | 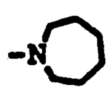 | Fp:72°C |
| 12 | F | $CHF_2$ | 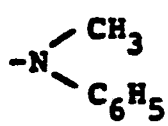 | Fp:78°C |

**Tabelle 2** (Fortsetzung)

| Bsp. Nr. | $R^1$ | $R^2$ | 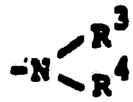 | physikalische Daten |
|---|---|---|---|---|
| 13 | F | $CHF_2$ | 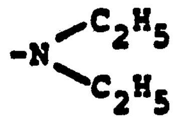 | Fp:50°C |
| 14 | Cl | $CHF_2$ | 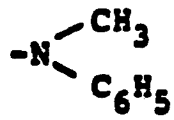 | Fp: 74°C |
| 15 | Cl | $CF_3$ | 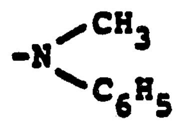 | Fp:115°C |
| 16 | Cl | $CF_3$ | 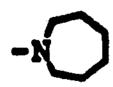 | Fp:70°C |
| 17 | Cl | $CF_3$ | 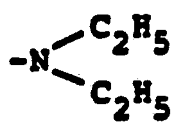 | Fp:39°C |
| 18 | Cl | $CF_3$ | 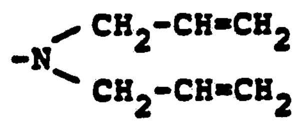 | Fp:66°C |
| 19 | Cl | $CF_3$ | 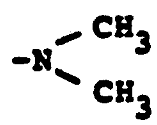 | Fp:58°C |
| 20 | Cl | $CF_3$ | 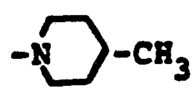 | Fp:72°C |
| 21 | Cl | $CF_3$ | 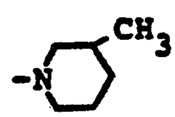 | $n_D^{20}$ =1,4908 |
| 22 | Cl | $CF_3$ | 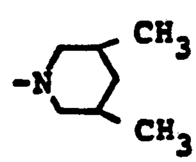 | $n_D^{20}$=1,4965 |
| 23 | Cl | $CF_3$ | 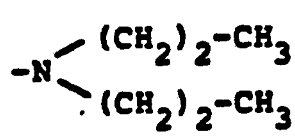 | $n_D^{20}$=1,4774 |

Tabelle 2 (Fortsetzung)

| Bsp. Nr. | $R^1$ | $R^2$ | 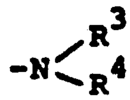 | physikalische Daten |
|---|---|---|---|---|
| 24 | Cl | $CF_3$ | 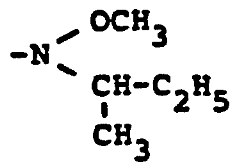 | $n_D^{20}$ =1,4715 |
| 25 | Cl | $CF_3$ | 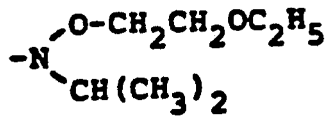 | $n_D^{20}$ =1,5664 |
| 26 | F | $CHF_2$ | 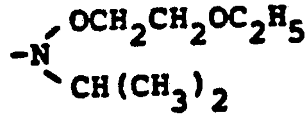 | Fp:54-56°C |
| 27 | F | $CHF_2$ | 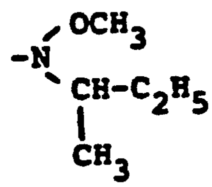 | $n_D^{20}$=1,4715 |
| 28 | F | $CHF_2$ | 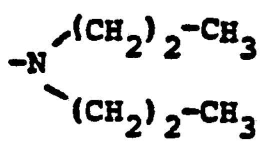 | $n_D^{20}$=1,4799 |
| 29 | Cl | $CF_3$ | 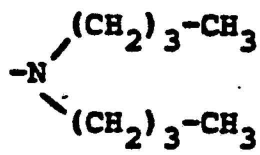 | $n_D^{20}$=1,4741 |
| 30 | Cl | $CF_3$ | 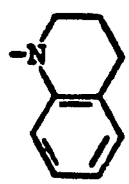 | Fp.: 60°C |
| 31 | F | $CF_3$ | 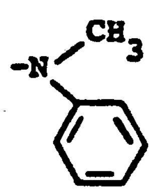 | Fp.: 54°C |
| 32 | F | $CF_3$ | 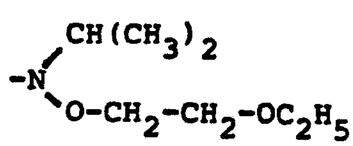 | $n_D^{20}$= 1,5953 |

**Herstellung der Ausgangsverbindungen:**

**Beispiel II-1**

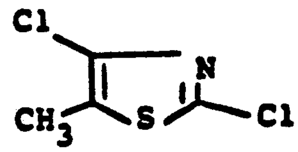

Eine Mischung aus 750 ml Phosphoroxychlorid, 157,2 g (1,2 Mol) 5-Methyl-2,4-thiazolidindion und 4 ml Dimethylformamid wird unter Rühren so lange unter Rückfluß erhitzt, bis die Gasentwicklung praktisch beendet ist (etwa 6 Stunden). Die erkaltete Reaktionsmischung wird anschließend unter gutem Rühren anteilweise auf 5 kg Eis ausgetragen. Dann wird dreimal mit je etwa einem Liter Methylenchlorid ausgeschüttelt, das Methylenchlorid im Vakuum abdestilliert und der Rückstand (187,3 g) destilliert. Man erhält bei 86°C/18 mbar 159,1 g (78,9 % der Theorie) 2,4-Dichlor-5-methylthiazol (in einer gaschromatographisch bestimmten Reinheit von 99,9 %) vom Siedepunkt 203° C (bei Atmosphärendruck).

**Beispiel II-2**

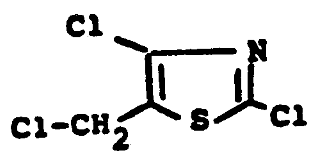

205 g (1,22 Mol) 2,4-Dichlor-5-methylthiazol werden in einer Chlorierungsapparatur (vgl. DE-OS-2 844 270, S. 18 und 23), unter UV-Bestrahlung mit einem Hg-Hochdruckstrahler unter Sieden (Sumpftemperatur anfangs 205°C) chloriert. Sobald die Sumpftemperatur 235°C erreicht hat, wird die Chlorierung abgebrochen: Umsatz etwa 80 %. Das Chlorierungsgemisch weist nach der gaschromatographischen Analyse die folgende Zusammensetzung auf:

19,5 % 2,4-Dichlor-5-methylthiazol
79,0 % 5-Chlormethyl-2,4-dichlorthiazol
1,5 % 2,1-Dichlor-5-(dichlormethyl)-thiazol.

Die fraktionierte Destillation an einer Füllkörperkolonne von 30 cm wirksamer Länge, gefüllt mit Glasringen von 2 mm Durchmesser und 2 mm Länge, liefert beim Kp 118 - 119°C/20 mbar 145 g (72 % der Theorie, bezogen auf den Umsatz) 5-Chlormethyl-2,4-dichlorthiazol; vom Brechungsindex $n_D^{20} = 1,5835$; GC-Reinheit: 98,5 %.

**Beispiel II-3**

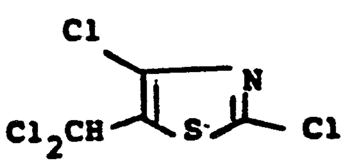

Zu einer Mischung aus 59 g (0,35 Mol) 2,1-Dichlor-5-methylthiazol und 450 ml Tetrachlormethan leitet man im Verlauf von 6 Stunden bei Rückflußtemperatur (ca. 80°C) 100 g (1,41 Mol) Chlor ein. Gemäß gaschromatographischer Analyse besteht das Reaktionsgemisch aus:

2,0 % 5-Chlormethyl-2,4-dichlorthiazol
89,4 % 2,4-Dichlor-5-(dichlormethyl)-thiazol
7,9 % 2,4-Dichlor-5-(trichlormethyl)-thiazol

Durch fraktionierte Destillation an einer Füllkörperkolonne von etwa 30 cm Länge erhält man als Hauptlauf bei Kp 122 - 125°C/20 mbar 48,5 g 2,4-Dichlor-4-(dichlormethyl)-thiazol in einer GC-Reinheit von 94,0 %. Ausbeute (bezogen auf 100 % Produkt): 54,8 % der Theorie.

**Beispiel II-4**

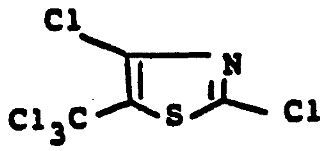

In 1.008 g (6 Mol) 2,4-Dichlor-5-methylthiazol gelöst in 600 ml Tetrachlormethan werden bei 200 bis 210°C im Verlauf von 6 Stunden 340 g Chlor und anschließend zwischen 210 und 240°C im Verlauf von 7 Stunden weitere 860 g Chlor (insgesamt 1.200 g = 16,9 Mol) eingeleitet. Das Gaschromatogramm des Rohgemisches zeigt, daß von den vier möglichen 2,4-Dichlorthiazolen (5-Methyl-, 5-Chlormethyl-, 5-Dichlormethyl- und 5-Trichlormethyl-) nur 2,4-Dichlor-5-(trichlormethyl)-thiazol vorhanden ist. Die destillative Aufarbeitung liefert bei 136 - 137°C/19 mbar 773 g (49,1 % der Theorie, bezogen auf die eingesetzte Chlormenge) 2,4-Dichlor-5-(trichlormethyl)-thiazol. GC-Reinheit: 97,1 %.

**Beispiel II-5**

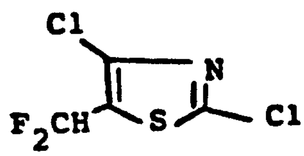

430 g (1,8 Mol) 2,4-Dichlor-5-dichlornethyl-1,3-thiazol werden mit 650 ml wasserfreier Flußsäure im VA-Autoklaven bei 137 - 140°C/18 - 22 bar fluoriert. Der entstehende Chlorwasserstoff wird laufend entspannt. Nach Ende der Reaktion wird die überschüssige Flußsäure im Vakuum bei Raumtemperatur abgezogen, der Ruckstand wird auf Eiswasser gegeben, in Dichlormethan aufgenonmen, über Natriumsulfat getrocknet und destilliert.

Man erhält 275 g (74,3 % der Theorie) 2,4-Dichlor-5-difluormethyl-1,3-thiazol, Kp 12 mbar/65 - 66°C; $n^{20}_D$ = 1,5070; sowie 30 g höher fluorierte Anteile.

**Beispiele II-6 und II-7**

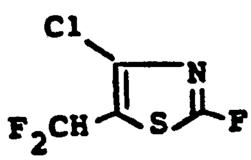

und

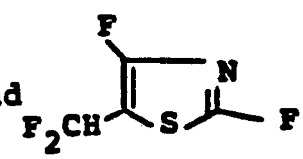

230 g (1,12 Mol) 2,4-Dichlor-5-difluormethyl-1,3-thiazol werden mit 131 g (2,25 Mol) Kaliumfluorid in 339 ml Tetramethylensulfon 3 Stunden bei 160°C gerührt. Anschließend wird das fluorierte Produkt bis zum Siedepunkt des Tetramethylensulfons im Vakuum abdestilliert.

Durch Redestillation erhält man:

76 g (40 % der Theorie) 2,4-Difluor-5-difluormethyl-1,3-thiazol vom Siedepunkt Kp: 108 - 9°C; Brechungsindex $n^{20}_D$ = 1,4108

und

47 g (22,4 % der Theorie) 2-Fluor-4-chlor-5-difluormethyl-1,3-thiazol vom Siedepunkt Kp: 141 - 3°C; Brechungsindex $n^{20}_D$ = 1,4528

sowie

40 g Ausgangsverbindung.

**Beispiele II-8 und II-9**

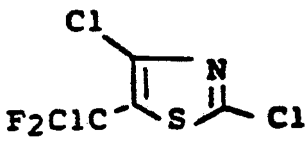 und 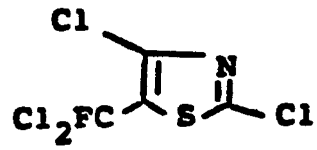

227 g (0,836 Mol) 2,4 -Dichlor-5-trichlormethyl-1,3-thiazol werden mit 200 ml wasserfreier Flußsäure im VA-Autoklaven bei 50°C/3 - 8 bar fluoriert. Der intstehende Chlorwasserstoff wird laufend intspannt. Nach Ende der Reaktion, (ca. 4 Stunden) wird auf Raumtemperatur abgekühlt und die überschüssige Flußsäure im Vakuum bis 100 mbar abgezogen. Der Rückstand wird auf Eiswasser gegeben, in Dichlormethan aufgenommen, über Natriumsulfat getrocknet und destilliert.
Man erhält:

84 g (39,5 % der Theorie) 2,4-Dichlor-5-difluorchlormethyl-1,3-thiazol vom Siedepunkt Kp: 76 - 8°C/18 mbar; Brechungsindex $n^{20}_D = 1,5120$

und

67 g (33.7 % der Theorie) 2,4-Dichlor-5-dichlorfluormethyl-1,3-thiazol von Siedepunkt Kp: 105 - 107°C/18 mbar, Brechungsindex $n^{20}_D = 1,5539$

sowie

12 g Ausgangsverbindung.

Wird die Reaktion bei 60°C/5 bar durchgeführt, erhält man mit einer Ausbeute von 71 % der Theorie 2,4-Dichlor-5-difluorchlormethyl-1,3-thiazol.

**Beispiel II-10**

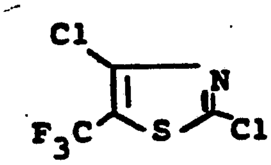

500 g (1,84 Mol) 2,4-Dichlor-5-trichlormethyl-1,3-thiazol werden mit 740 ml wasserfreier Flußsäure im VA-Autoklaven bei 120 - 140°C/25 - 30 bar 3 Stunden fluoriert. Der entstehende Chlorwasserstoff wird laufend entspannt. Nach Ende der Reaktion wird abgekühlt und die überschüssige Flußsäure im Wasserstrahlvakuum bis 100 mbar abgezogen. Der Rückstand wird auf Eiswasser gegeben, in Methylenchlorid aufgenommen, über Natriumsulfat getrocknet und destilliert.
Man erhält:

280 g (68,5 % der Theorie) 2,4-Dichlor-5-trifluormethyl-1,3-thiazol vom Siedepunkt Kp: 50°C/16 mbar. Brechungsindex $n^{20}_D = 1,4710$

sowie

63 g anfluorierte Verbindungen.

**Verwendungsbeispiele**

In dem folgenden Verwendungsbeispiel wurde die nachstehend aufgeführte Verbindung als Vergleichssubstanz eingesetzt:

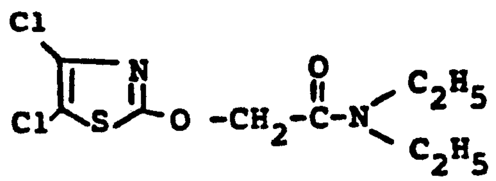

(A)

2-(4,5-Dichlor-1,3-thiazol-2-yloxy)-N,N-diethyl-acetamid (bekannt aus EP-OS-18 497).

**Beispiel A**

Pre-imergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffe pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der herbiziden Wirksamkeit bei vergleichbarer Nutzpflanzenselektivität gegenüber dem Stand der Technik zeigen bei diesem Test z. B. die Verbindungen gemäß folgender Herstellungsbeispiele: 12, 14, 15, 16, 17, 24 und 25.

**Patentansprüche** für die Vertragsstaaten: BE, CH, DE, FR, GB, IT, LI und NL

1. 4,5-Disubstituierte 1,3-Thiazol-2-yloxyacetamide der allgemeinen Formel (I)

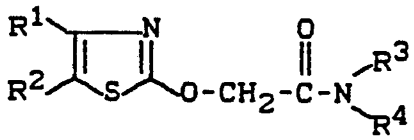

(I)

in welcher

$R^1$ für Fluor oder Chlor steht,

$R^2$ für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen steht und

$R^3$ und $R^4$ unabhängig voneinander für geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen (wobei die beiden letzgenannten Reste durch Alkylreste mit 1 bis 4 Kohlenstoffatomen substituiert sein können), für jeweils geradkettiges oder verzweigtes Alkoxy, Alkoxyalkylenoxy oder Alkoxyalkyl mit jeweils 1 bis 8 Kohlenstoffatomen in den einzelnen Alkyl- bzw. Alkylenteilen, für Halogenalkyl mit 1 bis 8 Kohlenstoffatomen und 1 bis 5 Halogenatomen für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen (wobei diese Reste substituiert sein können durch: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2

Kohlenstoffatomen und 1 bis 5 Halogenatomen, sowie Nitro), oder

$R^3$ und $R^4$ zusammen mit dem Stickstoffatom, an das sie gebunden sind, für einen gegebenenfalls einfach oder mehrfach, gleich oder verschieden substituierten, gesättigten oder ungesättigten, 5- bis 7-gliedrigen Heterocyclus stehen, (wobei diese Reste substiutiert sein können durch: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems, oder Dioxyalkylen mit 2 bis 3 Kohlenstoffatomen).

2. Verbindungen der allgemeinen Formel (I) gemäß Anspruch 1, dadurch gekennzeichnet, daß darin

$R^1$ für Fluor oder Chlor steht,

$R^2$ für Methyl, Trifluormethyl, Difluormethyl, Fluormethyl, Trichlormethyl, Dichlormethyl, Chlormethyl, Fluordichlormethyl oder Difluorchlormethyl steht und

$R^3$ und $R^4$ unabhängig voneinander für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, für gegebenenfalls ein- bis dreifach, gleich oder verschieden durch Methyl oder Ethyl substituiertes Cycloalkyl bzw. Cycloalkenyl mit 5 bis 7 Kohlenstoffatomen, für jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen (insbesondere Fluor, Brom und Chlor), für Benzyl sowie für gegebenenfalls ein- bis dreifach, gleich oder verschieden substituiertes Phenyl stehen, wobei als Substituenten besonders bevorzugt sind: Methyl, Ethyl, Methoxy, Methylthio, Trifluormethyl, Trifluormethoxy, Trifluormethylthio, Fluor, Chlor oder Nitro; oder

$R^3$ und $R^4$ zusanmen mit dem Stickstoffatom, an das sie gebunden sind, für einen gegebenenfalls ein- bis dreifach, gleich oder verschieden substituierten Heterocyclus der Formel

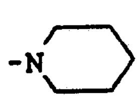 ; 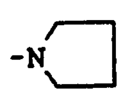 ; 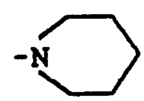 ;

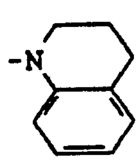 ; 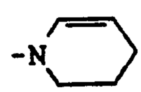 oder 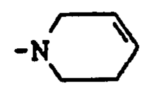

stehen, wobei als Substituenten besonders bevorzugt sind: Methyl, Ethyl und Phenyl.

3. Verfahren zur Herstellung von 4,5-disubstituierten 1,3-Thiazol-2-yloxyacetamiden der allgemeinen Formel (I) gemäß Anspruch 1,
dadurch gekennzeichnet, daß man 4,5-disubstituierte 1,3-Thiazole der Formel

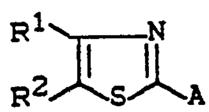 (II)

in welcher

$R^1$ und $R^2$ die in Anspruch 1 angegebene Bedeutung haben und

A für eine elektronenanziehende Abgangsgruppierung steht,

mit Glykolsäureamiden der Formel (III)

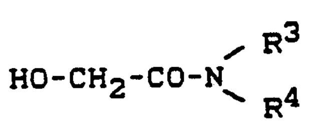 (III)

in welcher

$R^3$ und $R^4$ die in Anspruch 1 angegebene Bedeutung haben,

gegebenenfalls in Gegenwart eines Verdünnungsmittels und gegebenenfalls in Gegenwart eines Säurebindemittels sowie gegebenenfalls in Gegenwart eines Katalysators umsetzt.

4. Herbizide Mittel, gekennzeichnet durch einen Gehalt an mindestens einem 4,5-disubstituierten 1,3-Thiazol-2-yloxyacetamid der allgemeinen Formel gemäß Anspruch 1.

5. Verwendung von 4,5-disubstituierten 1,3-Thiazol-2-yloxyacetamiden der allgemeinen Formel (I) gemäß Anspruch 1 zur Bekämpfung von unerwünschtem Pflanzenwachstum.

6. Verfahren zur Herstellung von herbiziden Mitteln, dadurch gekennzeichnet, daß man 4,5-disubstituierte 1,3-Thiazol-2-yloxyacetamide der allgemeinen Formel (I) gemäß Anspruch 1 mit Streckmitteln und/oder

oberflächenaktiven Mitteln vermischt.

**Patentansprüche** für den Vertragsstaat: AT

1. Verfahren zur Herstellung von 4,5-disubstituierten 1,3-Thiazol-2-yloxyacetamiden der allgemeinen Formel (I)

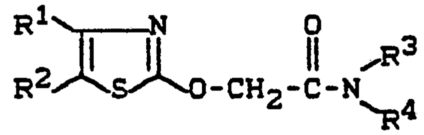

(I)

in welcher

$R^1$ für Fluor oder Chlor steht,

$R^2$ für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen (wie insbesondere Fluor, Chlor oder Brom) steht und

$R^3$ und $R^4$ unabhängig voneinander für geradkettiges oder verzweigtes Alkyl mit 1 bis 8 Kohlenstoffatomen, geradkettiges oder verzweigtes Alkenyl und Alkinyl mit jeweils 2 bis 8 Kohlenstoffatomen, für gegebenenfalls einfach oder mehrfach, gleich oder verschieden substituiertes Cycloalkyl oder Cycloalkenyl mit jeweils 3 bis 7 Kohlenstoffatomen (wobei die beiden letzgenannten Reste durch Alkylreste mit 1 bis 4 Kohlenstoffatomen substituiert sein können), für jeweils geradkettiges oder verzweigtes Alkoxy, Alkoxyalkylenoxy oder Alkoxyalkyl mit jeweils 1 bis 8 Kohlenstoffatomen in den einzelnen Alkyl- bzw. Alkylenteilen, für Halogenalkyl mit 1 bis 8 Kohlenstoffatomen und 1 bis 5 Halogenatomen (insbesondere Fluor, Chlor und Brom), für Aralkyl mit 6 bis 10 Kohlenstoffatomen im Arylteil und 1 bis 2 Kohlenstoffatomen im Alkylteil, sowie für gegebenenfalls einfach oder mehrfach, gleich oder verschieden substituiertes Aryl mit 6 bis 10 Kohlenstoffatomen stehen (wobei diese Reste substituiert sein können durch: Halogen, geradkettiges oder verzweigtes Alkyl, Alkoxy oder Alkylthio mit jeweils 1 bis 4 Kohlenstoffatomen, Halogenalkyl, Halogenalkoxy und Halogenalkylthio mit jeweils 1 bis 2 Kohlenstoffatomen und 1 bis 5 Halogenatomen, sowie Nitro), oder

$R^3$ und $R^4$ zusammen mit dem Stickstoffatom, an das sie gebunden sind, für einen gegebenenfalls einfach oder mehrfach, gleich oder verschieden substituierten, gesättigten oder ungesättigten, 5- bis 7-gliedrigen Heterocyclus stehen, (wobei diese Reste substiutiert sein können durch: geradkettiges oder verzweigtes Alkyl mit 1 bis 6 Kohlenstoffatomen, auch in Form eines anellierten Ringsystems, Aryl mit 6 bis 10 Kohlenstoffatomen, auch in Form eines anellierten Ringsystems, oder Dioxyalkylen mit 2 bis 3 Kohlenstoffatomen),

dadurch gekennzeichnet, daß man 4,5-disubstituierte 1,3-Thiazole der Formel

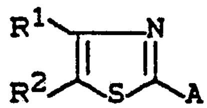

(II)

in welcher

$R^1$ und $R^2$ die oben angegebene Bedeutung haben und

A für eine elektronenanziehende Abgangsgruppierung steht,

mit Glykolsäureamiden der Formel (III)

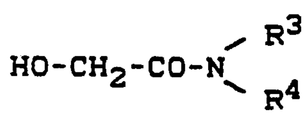

(III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels sowie gegebenenfalls in Gegenwart eines Katalysators umsetzt.

2. Herbizide Mittel, gekennzeichnet durch einen Gehalt an mindestens einem 4,5-disubstituierten 1,3-Thiazol-2-yloxyacetamid der allgemeinen Formel (I) gemäß Anspruch 1.

3. Verwendung von 4,5-disubstituierten 1,3-Thiazol-2-yloxyacetamiden der allgemeinen Formel (I) gemäß Anspruch 1 zur Bekämpfung von unerwünschtem Pflanzenwachstum.

4. Verfahren zur Herstellung von herbiziden Mitteln, dadurch gekennzeichnet, daß man 4,5-disubstituierte 1,3-Thiazol-2-yloxyacetamide der allgemeinen Formel (I) gemäß Anspruch 1 mit Streckmitteln und/oder oberflächenaktiven Mitteln vermischt.

**Claims** for the Contracting States: BE, CH, DE, FR, GB, IT, LI and NL

1. 4,5-Disubstituted 1,3-thiazol-2-yloxyacetamides of the general formula (I)

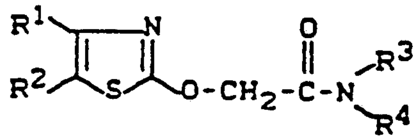

(I)

in which

$R^1$ represents fluorine or chlorine,

$R^2$ represents straight-chain or branched alkyl or halogenoalkyl each with 1 to 4 carbon atoms and, where appropriate, 1 to 9 identical or different halogen atoms, and

$R^3$ and $R^4$ independently of one another represent straight-chain or branched alkyl with 1 to 8 carbon atoms, straight-chain or branched alkenyl and alkinyl each with 2 to 8 carbon atoms, cycloalkyl or cycloalkenyl, each with 3 to 7 carbon atoms, which optionally have one or more identical or different substituents (suitable substituents for the latter two radicals being, in particular, alkyl radicals with 1 to 4 carbon atoms), straight-chain or branched alkoxy, alkoxyalkyleneoxy or alkoxyalkyl each with 1 to 8 carbon atoms in the individual alkyl or alkylene moieties, halogenoalkyl with 1 to 8 carbon atoms and 1 to 5 halogen atoms, aralkyl with 6 to 10 carbon atoms in the aryl moiety and 1 to 2 carbon atoms in the alkyl moiety, as well as aryl with 6 to 10 carbon atoms which optionally has one or more identical or different substituents (possible substituents being halogen, straight-chain or branched alkyl, alkoxy or alkylthio each with 1 to 4 carbon atoms, and halogenoalkyl, halogenoalkoxy and halogenoalkylthio each with 1 to 2 carbon atoms and 1 to 5 halogen atoms, as well as nitro), or

$R^3$ and $R^4$ together with the nitrogen atom to which they are bonded represent a saturated or unsaturated, 5- to 7-membered heterocyclic ring which optionally has one or more identical or different substituents, (possible substituents being straight-chain or branched alkyl with 1 to 6 carbon atoms, also in the form of a fused ring system, aryl with 6 to 10 carbon atoms, also in the form of a fused ring system, or dioxyalkylene with 2 to 3 carbon atoms).

2. Compounds of the general formula (I) according to Claim 1, characterised in that

$R^1$ represents fluorine or chlorine,

$R^2$ represents methyl, trifluoromethyl, difluoromethyl, fluoromethyl, trichloromethyl, dichloromethyl, chloromethyl, fluorodichloromethyl or difluorochloromethyl and

$R^3$ and $R^4$ independently of one another represent straight-chain or branched alkyl with 1 to 6 carbon atoms, straight-chain or branched alkenyl and alkinyl each with 2 to 6 carbon atoms, cycloalkyl or cycloalkenyl with 5 to 7 carbon atoms which are optionally mono- to tri-substituted by methyl or ethyl, the substituents being identical or different, alkoxy, alkoxyalkyleneoxy or alkoxyalkyl, each of which is straight-chain or branched and has 1 to 6 carbon atoms in the individual alkyl moieties, halogenoalkyl with 1 to 6 carbon atoms and 1 to 5 halogen atoms (especially fluorine, bromine and chlorine) benzyl as well as phenyl which optionally has one to three identical or different substituents, particularly preferred substituents being methyl, ethyl, methoxy, methylthio, trifluoromethyl, trifluoromethoxy, trifluoromethylthio, fluorine, chlorine or nitro; or

$R^3$ and $R^4$ together with the nitrogen atom to which they are bonded represent a heterocyclic ring of the formula

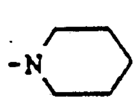 ; 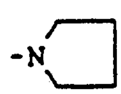 ; 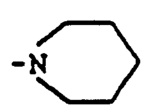 ;

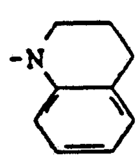 ; 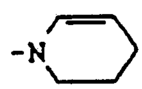 or 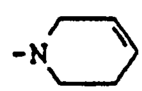

which optionally has one to three identical or different substituents, particularly preferred substituents being methyl, ethyl and phenyl.

3. Process for the preparation of 4,5-disubstituted 1,3-thiazol-2-yloxyacetamides of the general formula (I) according to Claim 1 characterised in that 4,5-disubstituted 1,3-thiazoles of the formula (II)

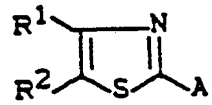

(II)

in which

$R^1$ and $R^2$ have the meaning indicated in Claim 1 and

A represents an electron-attracting leaving group are reacted with glycolic acid amides of the formula (III)

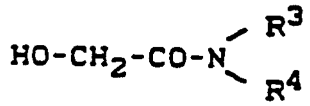

(III)

in which

$R^3$ and $R^4$ have the meaning indicated in Claim 1, if appropriate in the presence of a diluent and if appropriate in the presence of an acid acceptor as well as, if appropriate, in the presence of a catalyst.

4. Herbicidal agents, characterised in that they contain at least one 4,5-disubstituted 1,3-thiazol-2-yloxyacetamide of the general formula (I) according to Claim 1.

5. Use of 4,5-disubstituted 1,3-thiazol-2-yloxyacetamides of the general formula (I) according to Claim 1 for combating undesired plant growth.

6. A process for the preparation of herbicidal agents, characterised in that 4,5-disubstituted 1,3-thiazol-2-yloxyacetamides of the general formula (I) according to Claim 1 are mixed with extenders and/or surface-active agents.

**Claims** for the Contracting State: AT

1. Process for the preparation of 4,5-disubstituted 1,3-thiazol-2-yloxyacetamides of the general formula (I)

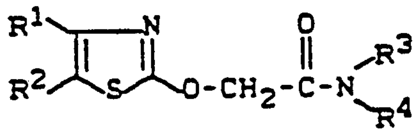

(I)

in which

$R^1$ represents fluorine or chlorine,

$R^2$ represents straight-chain or branched alkyl or halogenoalkyl each with 1 to 4 carbon atoms and, where appropriate, 1 to 9 identical or different halogen atoms, (such as, in particular, fluorine, chlorine or bromine) and

$R^3$ and $R^4$ independently of one another represent straight-chain or branched alkyl with 1 to 8 carbon atoms, straight-chain or branched alkenyl and alkinyl each with 2 to 8 carbon atoms, cycloalkyl or cycloalkenyl, each with 3 to 7 carbon atoms, which optionally have one or more identical or different substituents (suitable substituents for the two latter radicals being, in particular, alkyl radicals with 1 to 4 carbon atoms), straight-chain or branched alkoxy, alkoxyalkyleneoxy or alkoxyalkyl each with 1 to 8 carbon atoms in the individual alkyl or alkylene moieties, halogenoalkyl with 1 to 8 carbon atoms and 1 to 5 halogen atoms (especially fluorine, chlorine and bromine) aralkyl with 6 to 10 carbon atoms in the aryl moiety and 1 to 2 carbon atoms in the alkyl moiety, as well as aryl with 6 to 10 carbon atoms which optionally has one or more identical or different substituents (possible substituents being halogen, straight-chain or branched alkyl, alkoxy or alkylthio each with 1 to 4 carbon atoms, and halogenoalkyl, halogenoalkoxy and halogenoalkylthio each with 1 to 2 carbon atoms and 1 to 5 halogen atoms, as well as nitro), or

$R^3$ and $R^4$ together with the nitrogen atom to which they are bonded represent a saturated or unsaturated, 5- to 7-membered heterocyclic ring which optionally has one or more identical or different substituents, (possible substituents being straight-chain or branched alkyl with 1 to 6 carbon atoms, also in the form of a fused ring system, aryl with 6 to 10 carbon atoms, also in the form of a fused ring system, or dioxyalkylene with 2 to 3 carbon atoms), characterised in that 4,5-disubstituted 1,3-thiazoles of the formula

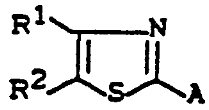

(II)

in which

$R^1$ and $R^2$ have the abovementioned meaning, and

A represents an electron-attracting leaving group are reacted with glycolic acid amides of the formula (III)

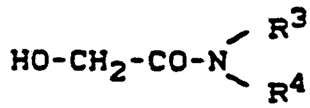

(III)

in which

$R^3$ and $R^4$ have the abovementioned meaning if appropriate in the presence of a diluent and if appropriate in the presence of an acid acceptor agent, as well as, if appropriate, in the presence of a catalyst.

2. Herbicidal agents, characterised in that they contain at least one 4,5-disubstituted 1,3-thiazol-2-yloxyacetamide of the general formula (I) according to Claim 1.

3. Use of 4,5-disubstituted 1,3-thiazol-2-yloxyacetamides of the general formula (I) according to Claim 1 for combating undesired plant growth.

4. A process for the preparation of herbicidal agents, characterised in that 4,5-disubstituted 1,3-thiazol-2-yloxyacetamides of the general formula (I) according to Claim 1 are mixed with extenders and/or surface-active agents.

**Revendications** pour les Etats Contractants: BE, CH, DE, FR, GB, IT, LI, NL

1. 1,3-thiazole-2-yloxyacétamides disubstitués en positions 4,5, de formule générale (I)

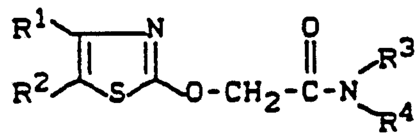

(I)

dans laquelle

$R^1$ représente le fluor ou le chlore,

$R^2$ représente un groupe alkyle ou halogénalkyle chacun à chaîne droite ou à chaîne ramifiée, avec 1 à 4 atomes de carbone et le cas échéant 1 à 9 atomes d'halogénes identiques ou différents et

$R^3$ et $R^4$ représentent indépendamment l'un de l'autre un groupe alkyle à chaîne droite ou à chaîne ramifiée ayant 1 à 8 atomes de carbone, un groupe alcényle et alcynyle à chaîne droite ou ramifiée ayant chacun 2 à 8 atomes de carbone, un groupe cycloalkyle ou cycloalcényle de 3 à 7 atomes de carbone portant éventuellement un ou plusieurs substituants identiques ou différents (les deux restes mentionnés en dernier lieu pouvant être substitués par des restes alkyle ayant 1 à 4 atomes de carbone), un groupe alkoxy, alkoxyalkylèneoxy ou alkoxyalkyle chacun à chaîne droite ou à chaîne ramifiée, avec 1 à 8 atomes de carbone dans les parties alkyle et alkylène individuelles, un groupe halogénalkyle ayant 1 à 8 atomes de carbone et 1 à 5 atomes d'halogènes, un groupe aralkyle de 6 à 10 atomes de carbone dans la partie aryle et de 1 ou 2 atomes de carbone dans la partie alkyle, ainsi qu'un reste aryle de 6 à 10 atomes de carbone portant éventuellement un ou plusieurs substituants identiques ou différents (ces restes pouvant être substitués par: un halogène, un groupe alkyle, alkoxy ou alkylthio à chaîne droite ou à chaîne ramifiée ayant chacun 1 à 4 atomes de carbone, un groupe halogénalkyle, halogénalkoxy et halogénalkylthio ayant chacun un ou deux atomes de carbone et 1 à 5 atomes d'halogènes, ainsi que le groupe nitro), ou bien

$R^3$ et $R^4$ forment conjointement avec l'atome d'azote auquel ils sont liés un hétérocycle pentagonal à heptagonal saturé ou non saturé portant éventuellement un ou plusieurs substituants identiques ou différents (les restes pouvant être substitués par: un radical alkyle à chaîne droite ou ramifiée ayant 1 à 6 atomes de carbone, également sous la forme d'un système de noyaux condensés, un radical aryle ayant 6 à 10 atomes de carbone, également sous la forme d'un système de noyaux condensés, ou un radical dioxyalkylène de 2 ou 3 atomes de carbone).

2. Composés de formule générale (I) suivant la revendication 1, caractérisés en ce que dans la formule

$R^1$ représente le fluor ou le chlore,

$R^2$ est un groupe méthyle, trifluorométhyle, difluorométhyle, fluorométhyle, trichlorométhyle,

dichlorométhyle, chlorométhyle, fluorodichlorométhyle ou difluorochlorométhyle et

$R^3$ et $R^4$ représentent, indépendamment l'un de l'autre, un groupe alkyle à chaîne droite ou à chaîne ramifiée ayant 1 à 6 atomes de carbone, un groupe alcényle et un groupe alcynyle à chaîne droite ou à chaîne ramifiée ayant chacun 2 à 6 atomes de carbone, un groupe cycloalkyle ou cycloalcényle de 5 à 7 atomes de carbone portant éventuellement 1 à 3 substituants méthyle ou éthyle identiques ou différents, un groupe alkoxy, alkoxyalkylèneoxy ou alkoxyalkyle chacun à chaîne droite ou à chaîne ramifiée, avec 1 à 6 atomes de carbone dans les parties alkyliques individuelles, un groupe halogénalkyle de i à 6 atomes de carbone et 1 à 5 atomes d'halogènes, (notamment fluor, brome et chlore), le groupe benzyle de même qu'un groupe phényle portant éventuellement 1 à 3 substituants identiques ou différents, et on apprécie alors particulièrement comme substituants: les radicaux méthyle, éthyle, méthoxy, méthylthio, trifluorométhyle, trifluorométhoxy, trifluorométhylthio, fluoro, chloro ou nitro; ou bien

$R^3$ et $R^4$ forment conjointement avec l'atome d'azote auquel il sont liés un hétérocycle portant le cas échéant 1 à 3 substituants identiques ou différents et répondant à la formule:

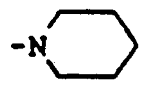 ; 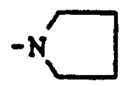 ; 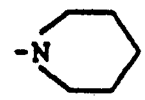 ;

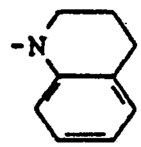 ; 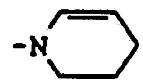 ou 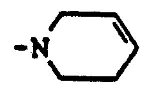

des substituants particulièrement appréciés étant les substituants méthyle, éthyle et phényle.

3. Procédé de préparation de 1,3-thiazole-2-yloxyacétamides disubstitués en positions 4,5 de formule générale (I) suivant la revendication 1,

caractérisé en ce qu'on fait réagir des 1,3-thiazoles disubstitués en positions 4,5 de formule (II)

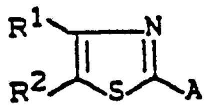 (II)

dans laquelle

$R^1$ et $R^2$ ont la définition indiquée dans la revendication 1 et

A est un groupe partant attirant les électrons, avec des amides d'acide glycolique de formule (III)

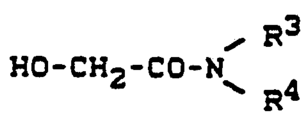 (III)

dans laquelle

$R^3$ et $R^4$ ont la définition indiquée dans la revendication 1,

éventuellement en présence d'un diluant et le cas échéant en présence d'un accepteur d'acide, ainsi que, le cas échéant, en présence d'un catalyseur.

4. Compositions herbicides, caractérisées par une teneur en au moins un 1,3-thiazole-2-yloxyacétamide disubstitué en positions 4,5 de formule générale (I) suivant la revendication 1.

5. Utilisation de 1,3-thiazole-2-yloxyacétamides disubstitués en positions 4,5, de formule générale (I) suivant la revendication 1, pour combattre la croissance non désirée de plantes.

6. Procédé de préparation de compositions herbicides, caractérisé en ce qu'on mélange des 1,3-thiazole-2-yloxyacétamides disubstitués en positions 4,5 de formule générale (I) suivant la revendication 1 avec des diluants et/ou des agents tensio-actifs.

**Revendications** pour l'Etat Contractant: AT

1. Procédé de préparation de 1,3-thiazole-2-yloxyacétamides disubstitués en positions 4,5 de formule générale (I)

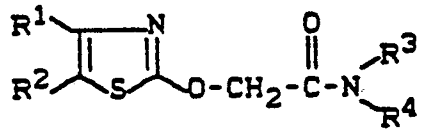

(I)

dans laquelle

$R^1$ représente le fluor ou le chlore,

$R^2$ représente un groupe alkyle ou halogénalkyle chacun à chaîne droite ou à chaîne ramifiée, avec 1 à 4 atomes de carbone et le cas échéant 1 à 9 atomes d'halogènes identiques ou différents (notamment le fluor, le chlore ou le brome) et

$R^3$ et $R^4$ représentent indépendamment l'un de l'autre un groupe alkyle à chaîne droite ou à chaîne ramifiée ayant 1 à 8 atomes de carbone, un groupe alcényle et alcynyle à chaîne droite ou ramifiée ayant chacun 2 à 8 atomes de carbone, un groupe cycloalkyle ou cycloalcényle de 3 à 7 atomes de carbone portant éventuellement un ou plusieurs substituants identiques ou différents (les deux restes mentionnés en dernier lieu pouvant être substitués par des restes alkyle ayant 1 à 4 atomes de carbone), un groupe alkoxy, alkoxyalkylèneoxy ou alkoxyalkyle chacun à chaîne ou à chaîne ramifiée, avec 1 à 8 atomes de carbone dans les parties alkyle et alkylène individuelles, un groupe halogénalkyle ayant 1 à 8 atomes de carbone et 1 à 5 atomes d'halogènes (notamment fluor chlore et brome), un groupe aralkyle de 6 à 10 atomes de carbone dans la partie aryle et de 1 ou 2 atomes de carbone dans la partie alkyle, ainsi qu'un reste aryle de 6 à 10 atomes de carbone portant éventuellement un ou plusieurs substituants identiques ou différents (ces restes pouvant être substitués par: un halogène, un groupe alkyle, alkoxy ou alkylthio à chaîne droite ou à chaîne ramifiée ayant chacun 1 à 4 atomes de carbone, un groupe halogénalkyle, halogénalkoxy et halogénalkylthio ayant chacun un ou deux atomes de carbone et 1 à 5 atomes d'halogènes, ainsi que le groupe nitro), ou bien

$R^3$ et $R^4$ forment conjointement avec l'atome d'azote auquel ils sont liés un hétérocycle pentagonal à heptagonal saturé ou non saturé portant éventuellement un ou plusieurs substituants identiques ou différents (les restes pouvant être substitués par: un radical alkyle à chaîne droite ou ramifiée ayant 1 à 6 atomes de carbone, également sous la forme d'un système de noyaux condensés, un radical aryle ayant 6 à 10 atomes de carbone, également sous la forme d'un système de noyaux condensés, ou un radical dioxyalkylène de 2 ou 3 atomes de carbone),

caractérisé en ce qu'on fait réagir des 1,3-thiazoles disubstitués en positions 4,5, de formule

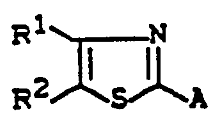

(II)

dans laquelle

$R^1$ et $R^2$ ont la définition indiquée ci-dessus et

A représente un groupe partant attirant les électrons,

avec des amides d'acide glycolique de formule (III)

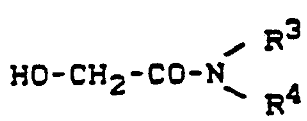

(III)

dans laquelle

$R^3$ et $R^4$ ont la définition indiquée ci-dessus,

éventuellement en présence d'un diluant et le cas échéant en présence d'un accepteur d'acide ainsi que, le cas échéant, en présence d'un catalyseur.

2. Compositions herbicides, caractérisées par une teneur en au moins un 1,3-thiazole-2-yloxyacétamide disubstitué en positions 4,5 de formule générale (I) suivant la revendication 1.

3. Utilisation de 1,3-thiazole-2-yloxyacétamides disubstitués en positions 4,5, de formule générale (I) suivant la revendication 1, pour combattre la croissance non désirée de plantes.

4. Procédé de préparation de compositions herbicides, caractérisé en ce qu'on mélange des 1,3-thiazole-2-yloxyacétamides disubstitués en positions 4,5 de formule générale (I) suivant la revendication 1 avec des diluants et/ou des agents tensio-actifs.